(19) Europäisches Patentamt
European Patent Office
Office européen des brevets

(11) Publication number: **0 108 539**
**B1**

(12) # EUROPEAN PATENT SPECIFICATION

(45) Date of publication of patent specification: **27.08.86**

(51) Int. Cl.⁴: **C 07 C 69/16,** C 07 C 67/37, C 07 C 67/293

(21) Application number: **83306416.5**

(22) Date of filing: **21.10.83**

(54) **Preparation of esters.**

(30) Priority: **30.10.82 GB 8231103**

(43) Date of publication of application:
**16.05.84 Bulletin 84/20**

(45) Publication of the grant of the patent:
**27.08.86 Bulletin 86/35**

(84) Designated Contracting States:
**BE DE FR GB NL**

(56) References cited:
**EP-A-0 025 702**

(73) Proprietor: **BP Chemicals Limited**
**Belgrave House 76 Buckingham Palace Road**
**London, SW1W 0SU (GB)**

(72) Inventor: **Cook, John**
**BP Chemicals Limited, Saltend**
**Hedon, Hull, HU12 8DS (GB)**
Inventor: **Drury, David James, BP Chemicals Limited**
**Belgrave House, 76 Buckingham Palace Road**
**London, SW1W OSU (GB)**

(74) Representative: **Crack, Richard David et al**
**c/o The British Petroleum Company plc Patents Division Chertsey Road**
**Sunbury-on-Thames Middlesex, TW16 7LN (GB)**

**EP 0 108 539 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for the preparation of ethylidene diacetate by reaction of methyl acetate or dimethyl ether with carbon monoxide and hydrogen.

Ethylidene diacetate is a valuable organic chemical since it can be converted to vinyl acetate and acetic acid by thermal cracking (see Kirk-Othmer "Encyclopaedia of Chemical Technical Technology" 2nd edition, Vol. 21 page 321) or alternatively can be pyrolysed to yield acetic anhydride and acetaldehyde (see above reference Vol. 8 page 410).

The conversion of methyl acetate to ethylidene diacetate by reaction with carbon monoxide and hydrogen in the presence of a Group VIII noble metal catalyst with a manganese or rhenium compound as a co-catalyst has been previously described in German DOS 2941232. The same reaction has also been described in European Patent No. 0,035,860 using a supported palladium compounds as catalyst.

Further, European patent application No. 025702 discloses the reaction of methyl acetate or dimethyl ether with hydrogen and carbon monoxide to form ethylidene diacetate in the presence of a catalyst system which can contain cobalt, a trivalent nitrogen or phosphorus compound and a bromide or iodide.

The trivalent nitrogen or phosphorus compound is described as a promoter and is said to be capable of forming a coordination bond with the cobalt of the catalyst. The specification does not explicitly say that the nitrogen or phosphorus is quaternised, although this may in some instances be inferred, since some of the compounds described as the source of iodide or bromide are known quaternising agents. However all these latter described compounds are halogen-containing.

It has now been found that the reaction can be significantly improved by operating with certain ratios of iodide to cobalt and with low quantities of halide or by including in the catalyst system a halide-free quaternising agent.

Thus, according to one aspect of the present invention a process for the preparation of ethylidene diacetate comprises reacting at elevated temperature methyl acetate or dimethyl ether with carbon monoxide and hydrogen in the presence of an effective amount of a catalyst system comprising cobalt and, as a promoter, (i) a trivalent nitrogen or phosphorus compound and a halide-free quaternising agent capable of quaternising the nitrogen or phosphorus under the reaction conditions (ii) an effective amount of a halide selected from iodide or bromide and where the atomic ratio of halide to cobalt is less than 5:1.

The benefit of using a ratio of halide to cobalt of less than 5:1 and using a halide-free quaternising agent is that the selectivity to ethylidene diacetate is increased. However according to one embodiment the process can be effected to coproduce significant quantities of acetic anhydride.

The terms iodide and bromide are intended to include iodine and bromine respectively. Unless the context clearly requires otherwise, the terms alkyl and aryl in the specification are intended to include aralkyl and alkaryl respectively.

The cobalt may be added in any convenient form, e.g. in the zero valent state or in any higher valent form. Thus, the cobalt may be added as the elemental metal in finely divided form, or as the carbonate, oxide, hydroxide, bromide, iodide, chloride, lower alkoxide such as methoxide, phenoxide, or carboxylate wherein the carboxylate anion is derived from an alkanoic acid of 1 to 20 carbon atoms. Similarly, complexes of the metal can be employed, for example, the cobalt may be coordinated with one or more ligands selected from carbon monoxide, halides, phosphines, arsines and trivalent nitrogen compounds. Suitably, cobalt may be added as a halide, a carboxylate salt, a carbonyl or a carbonyl halide.

The amount of catalyst can be from 100 to 10,000 ppm by wt. of cobalt based on the wt. of reaction charge preferably 1000 to 10,000 ppm.

The amount of halide present should be sufficient to effect promotion of the coablt and, as stated above, the atomic ratio of halide to cobalt is less than 5:1, but preferably should not exceed 100,000 ppm preferably 50,000 ppm more preferably less than 20,000 pm based on the weight of reaction charge.

According to another aspect of the present invention a process for the preparation of ethylidene diacetate comprises reacting at elevated temperature methyl acetate or dimethyl ether with carbon monoxide and hydrogen in the presence of an effective amount of a catalyst system comprising cobalt and, as promoter quaternary nitrogen or phosphorus and a halide selected from iodide or bromide and where the atomic ratio of halide to cobalt is less than 5:1 and the amount of halide present does not exceed 100,000 parts per million based on the weight of reaction charge.

The quaternary nitrogen or phosphorous may be preformed by reacting the quaternisable nitrogen or phosphorus compound with the halide-free quaternising agent in a separate step.

Suitable trivalent nitrogen compounds include heterocyclic amines such as pyridine, picoline, quinoline, hydroxyquinoline, methylquinoline, pyrrole, pyrrolidine, pyrrolidone and the like or an imidazole, such as imidazole, methylimidazole and the like. Suitable trivalent phosphorus compounds are of formula:—

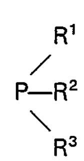

wherein $R^1$, $R^2$ and $R^3$ which may be the same or

different are alkyl groups of up to 20, preferably from 1 to 8 carbon atoms or monocyclic aryl groups, or $R^3$ may be the group:

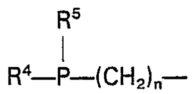

wherein $R^4$ and $R^5$ are each a monocyclic aryl group or an alkyl group and n is zero or an integer in the range 1 to 20. Suitable compounds having the above formula include trimethylphosphine, tripropylphosphine and triphenylphosphine.

Preferably the quaternizing agent is an aryl or an alkyl ester of a sulphonic acid. Suitable agents are alkyl or aryl sulphonates for example those of formula

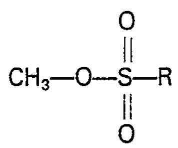

where R is hydrogen, an alkyl or aryl group or an alkoxy or aryloxy group, or F or $CF_3$. Conveniently the R group contains 1 to 12 carbon atoms. Examples are methyl p-toluene sulphonate, dimethyl sulphate, methyl sulphonic acid, and methyl fluorosulphonic acid.

By halide-free is meant not containing chlorine, bromine or iodine.

Although the molar ratio of the trivalent nitrogen or phosphorus to the quaternising agent may vary over a wide range, it may suitably be in the range from 0.1:1 to 10:1. It has been found that the catalyst efficiency increases as the ratio approaches 1:1 from either direction. Accordingly, a molar ratio of about 1:1 e.g. from 0.5:1 to 1.5:1 is preferred.

Suitable quaternary nitrogen-containing compounds are heterocyclic aromatic compounds for example those containing N - methylpyridinium, N,N - dimethylimidazolinium, N - methyl - 3 - picolinium, N - methyl - 3,4 - lutidinium and N - methylquinolinium conveniently as the tosylate.

Suitable quaternary phosphorus-containing compounds are those containing tetramethyl phosphonium, ethyltrimethyl phosphonium, methyltripropyl phosphonium or methyltriphenyl phosphonium.

Preferably the ratio of moles of quaternising agent to gm atoms of cobalt is at least 5:1 more preferably from 20:1 to 50:1 and can exceed 50:1.

A copromoter in the form of a lower monocarboxylic acid may be employed with advantage. The monocarboxylic acid suitably corresponds to the acid anhydride produced in the reaction. For example, using methyl acetate as feed, the monocarboxylic acid employed is preferably acetic acid.

The elevated temperature employed may suitably be in the range from 50 to 350°C, preferably from 80 to 200°C, and pressures can be from 27 to 170 Bar preferably between 61 and 116 Bar. The ratio of the partial pressure of carbon monoxide to hydrogen may suitably be in the range from 20:1 to 1:20. High ratios of $CO:H_2$ favour acetic anhydride formation over ethylidene diacetate, lower ratios for example lower than 5:1 such as from 5:1 to 1:5 favour formation of ethylidene diacetate.

The process may be carried out in the liquid phase or in the vapour phase. In the case of both liquid and vapour phase operation, the catalyst and optionally also the promoter combination may be supported, ie they may be dispersed on a conventional support material, such as for example alumina, silica, silica/alumina, zeolites, clays, titania or zirconia. The catalyst and optionally the promoter may be applied to the support in conventional manner, eg by impregnation from solution. The catalyst concentration on the support may suitably be in the range from 0.01 to 10% by weight.

The process of the invention may be carried out batchwise or continuously.

The invention is illustrated by the following examples;

In all the following Examples all the reactants and products except the CO and hydrogen were in the liquid phase and the catalyst and promoter were employed in solution.

Example 1

Methyl acetate (35 g), acetic acid (15 g) as copromoter, methyl p-toluenesulphonate (10 g) as quaternising agent, $CO_2(CO)_8$ (0.2 g), 1-methyimidazole (2.3 g) as the trivalent nitrogen compound and sodium iodide (0.5 g) (iodide:cobalt atomic ratio 3:1) were charged to a stainless steel autoclave which was pressured to about 7 Bar $H_2$ and to a further 69 Bar with CO at room temperature. This vessel was then heated and stirred for one hour at 130°C. At this temperature the initial total pressure was approximately 89 Bar.

Gas Chromatography (G.C.) analysis of the reaction mixture showed it to contain 0.6 g of acetic anhydride and 1.0 g of ethylidene diacetate.

Example 2

Example 1 was repeated except that 34 Bar $H_2$ and 61 Bar CO were used, and the autoclave was stirred for forty minutes at 150°C (the initial total pressure in the reactor was approximately 106 Bar).

Gas Chromatography (G.C.) analysis of the reaction mixture showed it to contain 0.1 g of acetic anhydride and 1.9 of ethylidene diacetate.

This example, when compared with Example 1, illustrates that a higher yield of ethylidene diacetate is favoured by higher hydrogen partial pressures.

Example 3

(Example 3 illustrates the reaction in the absence of acetic acid as copromoter.)

A mixture of methyl acetate (50 g) methyl

p-toluenesulphonate (10 g), $CO_2(CO)_8$ (0.2 g), 1-methylimidazole (2.3 g) and sodium iodide (0.5 g) was charged to a stainless steel autoclave and pressured with $H_2$ (34 Bar) and CO (61 Bar) at room temperature.

The vessel was stirred for 40 minutes at 150°C. At the reaction temperature the initial total pressure was approximately 109 Bar.

Gas Chromatography (G.C.) analysis of the reaction mixture showed it to contain 0.1 g of acetic anhydride and 0.2 g of ethylidene diacetate.

Example 4

A stainless steel autoclave was charged with a mixture of acetic acid (40 g), dimethyl ether (3.2 g), methyl p-toluenesulphonate (12 g), $CO_2(CO)_8$ (0.4 g), 1-methylimidazole (4 g) and sodium iodide (0.5 g). (Iodide:Cobalt atomic ratio (1.5:1).

A mixture of 34 Bar $H_2$ and 68 Bar CO was then pressed into the autoclave at room temperature. The vessel was stirred for one hour at 150°C. At this temperature the initial total pressure in the reactor was approximately 119 Bar.

Gas Chromatography (G.C.) analysis of the reaction mixture showed it to contain 1.2 g of methyl acetate, 0.2 g of acetic anhydride and 1.7 g of ethylidene diacetate.

Comparative Example A

Methyl acetate (35 g), acetic acid (15 g), $CO_2(CO)_8$ (0.2 g), 1-methylimidazole (2.3 g) and sodium iodide (0.5 g); (iodide:cobalt ratio 3:1) were charged to a stainless steel autoclave which was pressured to about 8 Bar $H_2$ and to about 67 Bar with CO at room temperature. This vessel was then heated and stirred for one hour at 200°C.

Gas Chromatography (G.C.) analysis of the reaction mixture showed it to contain a trace of acetic anhydride but no ethylidene diacetate.

This example demonstrates the use of a catalyst system containing an iodide to cobalt ratio of 3:1 in the absence of a quaternising agent and shows the yield of ethylidene diacetate is greatly reduced by the absence of the quaternising agent.

Comparative Example B

A stainless steel autoclave was charged with a mixture of methyl acetate (35 g), acetic acid (15 g) methyl iodide (8 g), $CO_2(CO)_8$ (0.3 g) and 1-methylimidazole (4.8 g). The ratio of I to cobalt was 32:1 and the methyl iodide to imidazole was 1:1.

A mixture of 34 Bar $H_2$ 61 Bar CO was then pressed into the autoclave at room temperature. The vessel was stirred for one hour at 200°C. At this temperature the initial total pressure in the reactor was approximately 109 Bar.

Gas Chromatography (G.C.) analysis of the reaction mixture showed it to contain 4.7 g of acetic anhydride and 0.4 g of ethylidene diacetate.

This example demonstrates the low selectivity to ethylidene diacetate obtained using an iodide-containing quaternising agent with the consequence that the catalyst system has an I:Co ratio of 32:1.

**Claims**

1. A process for the preparation of ethylidene diacetate which process comprises reacting at a temperature in the range of 50 to 350°C methyl acetate or dimethyl ether with carbon monoxide and hydrogen in the presence of an effective amount of a catalyst system comprising cobalt and, as promoter, nitrogen or phosphorus and a halide selected from iodide and bromide and where the atomic ratio of halide to cobalt is less than 5:1 characterised in that either (i) the nitrogen or phosphorus is quaternary nitrogen or phosphorus and the amount of halide does not exceed 100,000 parts per million based on the weight of reaction charge or (ii) the nitrogen or phosphorus is provided by a trivalent nitrogen or phosphorus compound, there also be being present a halide-free quaternising agent capable of quaternising the trivalent nitrogen or phosphorus under the reaction conditions.

2. A process as claimed in claim 1 characterised in that the quaternisation of the trivalent nitrogen or phosphorus with the halide-free quaternising agent is carried out before the reaction.

3. A process as claimed in claim 1 characterised in that the halide-free quaternising agent is a halide-free alkylating agent.

4. A process as claimed in claim 4 characterised in that the halide-free alkylating agent is an alkyl or aryl ester of a sulphonic acid.

5. A process as claimed in claim 1 characterised in that the ratio of moles of trivalent nitrogen or phosphorus compound to quaternising agent is from 0.1:1 to 10:1.

6. A process as claimed in claim 1 characterised in that the ratio of moles of quaternising agent to gm atoms of cobalt is at least 5:1.

7. A process as claimed in claim 1 characterised in that the molar ratio of carbon monoxide:hydrogen is from 20:1 to 1:20.

**Patentansprüche**

1. Verfahren zur Herstellung von Ethylidendiacetat, wobei das Verfahren die Umsetzung bei einer Temperatur in dem Bereich von 50 bis 350°C von Methylacetat oder Dimethylether mit Kohlenmonoxyd und Wasserstoff in der Gegenwart einer wirksamen Menge eines Katalysatorsystems umfaßt, das Kobalt und als Promotor Stickstoff oder Phosphor und ein Halogenid, ausgewählt unter Iodid und Bromid, enthält und worin das atomare Verhältnis von Halogenid zu Kobalt geringer als 5:1 ist, dadurch gekennzeichnet, daß entweder (i) der Stickstoff oder Phosphor quaternärer Stickstoff oder Phosphor ist und die Menge des Halogenides nicht 100.000 Teile pro Million bezogen auf das Gewicht der Reaktionscharge überschreitet oder (ii) der Stickstoff oder Phosphor durch eine dreiwertige Stickstoff- oder Phosphorverbindung zur Verfügung gestellt wird,

wobei dort auch ein Halogenidfreies quaternisierendes Mittel, das zur Quaternisierung des dreiwertigen Stickstoffs oder Phosphors unter den Reaktionsbedingungen fähig ist, vorhanden ist.

2. Verfahren nach Anspruch 1, dadurch gekennzeichnet, daß die Quaternisierung des dreiwertigen Stickstoffs oder Phosphors mit dem Halogenid-freien quaternisierenden Mittle vor der Umsetzung durchgeführt wird.

3. Verfahren nach Anspruch 1, dadurch gekennzeichnet, daß das Halogenid-freie quaternisierende Mittel ein Halogenid-freies alkylierendes Mittel ist.

4. Verfahren nach Anspruch 3, dadurch gekennzeichnet, daß das Halogenid-freie alkylierende Mittel ein Alkyl- oder Arylester einer Sulfonsäure ist.

5. Verfahren nach Anspruch 1, dadurch gekennzeichnet, daß das Verhältnis der Mole der dreiwertigen Stickstoff- oder Phosphorverbindung zu dem quaternisierenden Mittel von 0,1:1 bis 10:1 beträgt.

6. Verfahren nach Anspruch 1, dadurch gekennzeichnet, daß das Verhältnis der Mole des quaternisierenden Mittels zu den Gramatomen von Kobalt mindestens 5:1 beträgt.

7. Verfahren nach Anspruch 1, dadurch gekennzeichnet, daß das molare Verhältnis von Kohlenmonoxyd:Wasserstoff von 20:1 bis 1:20 beträgt.

**Revendications**

1. Procédé pour la préparation de diacétate d'éthylidène, ce procédé comprenant la réaction, à une température comprise dans l'intervalle de 50 à 350°C, de l'acétate de méthyle ou de l'étheroxyde de diméthyle avec le monoxyde de carbone et l'hydrogène en présence d'une quantité efficace d'un système de catalyseur comprenant du cobalt et, à titre de promoteur, de l'azote ou du phosphore et un halogénure choisi parmi un iodure et un bromure, le rapport entre atomes d'halogénure et de cobalt étant inférieur à 5:1, procédé caractérisé en ce que (i) l'azote ou le phosphore est de l'azote ou du phosphore quaternaire, et la quantité d'halogénure n'excède pas 100 000 parties par million sur la base du poids de la charge de réaction, ou (ii) l'azote ou le phosphore est fourni par un composé d'azote ou de phosphore trivalent, et il y a également présence d'un agent de quaternisation dépourvu d'halogénure et capable de quaterniser, dans les conditions de réaction, l'azote ou le phosphore trivalent.

2. Procédé selon la revendication 1, caractérisé en ce que l'on effectue avant la réaction la quaternisation de l'azote ou du phosphore trivalent à l'aide de l'agent de quaternisation dépourvu d'halogénure.

3. Procédé selon la revendication 1, caractérisé en ce que l'agent de quaternisation dépourvu d'halogénure est un agent d'alkylation dépourvu d'halogénure.

4. Procédé selon la revendication 3, caractérisé en ce que l'agent d'alkylation dépourvu d'halogénure est un ester d'alkyle ou d'aryle d'un acide sulfonique.

5. Procédé selon la revendication 1, caractérisé en ce que le rapport entre les moles du composé d'azote ou de phosphore trivalent et de l'agent de quaternisation se situe entre 0,1:1 et 10:1.

6. Procédé selon la revendication 1, caractérisé en ce que le rapport des moles de l'agent de quaternisation aux atomes-grammes de cobalt est au moins égal à 5:1.

7. Procédé selon la revendication 1, caractérisé en ce que le rapport molaire du monoxyde de carbone à l'hydrogène se situe entre 20:1 et 1:20.